(19) Europäisches Patentamt
European Patent Office
Office européen des brevets

(11) Veröffentlichungsnummer: **0 596 458 A1**

(12) # EUROPÄISCHE PATENTANMELDUNG

(21) Anmeldenummer: **93117746.3**

(22) Anmeldetag: **02.11.93**

(51) Int. Cl.[5]: **C07C 43/13**, C23C 16/18, C07C 215/18

(30) Priorität: **06.11.92 DE 4237522**

(43) Veröffentlichungstag der Anmeldung: **11.05.94 Patentblatt 94/19**

(84) Benannte Vertragsstaaten: **CH DE FR GB LI NL**

(71) Anmelder: **HOECHST AKTIENGESELLSCHAFT**
**Brüningstrasse 50**
**D-65929 Frankfurt am Main(DE)**

(72) Erfinder: **Herrmann, Wolfgang A., Prof. Dr.**
**Gartenstrasse 69**
**D-85354 Freising(DE)**
Erfinder: **Huber, Norbert, DCh.**
**Rosenweg 11**
**D-85386 Dietersheim bei Eching(DE)**

(54) **Mehrfunkfionelle Tertiäralkohole, Verfahren zu deren Herstellung und ihre Verwendung für Precursoren zur Auftragung von Erdalkalimetalloxiden nach dem Verfahren der chemical vapour deposition.**

(57) Mehrfunktionelle Tertiäralkohole, Verfahren zu deren Herstellung und ihre Verwendung für Precursoren zur Auftragung von Erdalkalimetalloxiden nach dem Verfahren der chemical vapor deposition.

Die Erfindung betrifft mehrfunktionelle Tertiäralkohole, die als Precursoren für die Auftragung von Metalloxiden nach dem Verfahren der chemical vapor deposition geeignet sind. Sie besitzen die allgemeine chemische Formel:

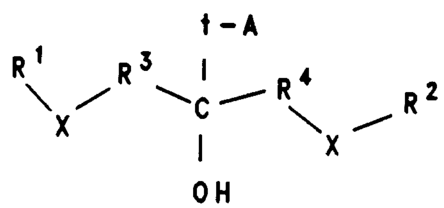

(I)

in der

| | |
|---|---|
| t-A | eine tertiäre Alkylgruppe wie t-Butyl, t-Amyl-, t-Hexyl- oder t-Heptyl-; |
| $R^1$ und $R^2$ | einen geradkettigen oder verzweigten Alkylrest mit 1 bis 7 C-Atomen oder die Gruppe $-[(CH_2)_nO-]_mR^5$ in der n und m unabhängig voneinander eine ganze Zahl von 1 bis 3 und $R^5$ eine geradkettige oder verzweigte Alkylgruppe mit 1 bis 5 C-Atomen bedeuten, |
| $R^3$ und $R^4$ | einen geradkettigen oder verzweigten Alkylenrest mit 1 bis 4 C-Atomen; und |
| X | eine heteroatomhaltige Gruppe wie -O-, >N-$CH_3$, >N-$C_2H_5$ oder >N-$C_3H_7$ bedeuten. |

Die Erfindung betrifft auch flüchtige Komplexe von Erdalkalimetallen mit den mehrfunktionellen Tertiäralkoholen.

EP 0 596 458 A1

Mehrfunktionelle Tertiäralkohole, Verfahren zu deren Herstellung und ihre Verwendung für Precursoren zur Auftragung von Erdalkalimetalloxiden nach dem Verfahren der chemical vapor deposition.

Die vorliegende Erfindung betrifft neue Precursoren für die Auftragung von Schichten aus Erdalkalimetalloxiden nach dem Verfahren der chemical vapor deposition (CVD-Verfahren = chemische Abscheidung aus der Gasphase).

Nach diesem Verfahren werden Materialien verdampft, gelangen in gasförmigem Zustand an ein Substrat und schlagen sich darauf unter gleichzeitiger Zersetzung nieder.

Metall-Alkoxide, auch Metall-Alkoholate genannt, haben sich als Ausgangsverbindungen für oxidische Materialien für das CVD-Verfahren gut bewährt (siehe Caulton et al., Chem. Rev., Jg. 1990, S. 969-995). Die Entwicklung bariumhaltiger keramischer Hochtemperatur-Supraleiter hat deshalb in den zurückliegenden Jahren zu einem stetig zunehmenden Interesse an den dahin kaum erforschten Barium-Alkoxiden $Ba(OR)_2$ bzw. -Aryloxiden $Ba(OAr)_2$ geführt. Trotz einiger Fortschritte wurde aber das Ziel flüchtiger und für die bestimmungsgemäßen Einsatzzwecke gleichzeitig hinreichend thermisch stabiler Verbindungen nicht erreicht. Darüber hinaus dürfen keine Elemente wie Fluor oder Silicium in den Verbindungen enthalten sein, da sonst störende fluorid- bzw. siliziumhaltige Komponenten, hauptsächlich $BaF_2$ und $SiO_2$, abgeschieden werden.

Aufgabe der Erfindung war es, organische Komplexliganden zu entwickeln, die es ermöglichen, Schwermetallionen, insbesondere Bariumionen, in flüchtige, gleichzeitig aber thermisch hinreichend stabile Verbindungen zu überführen, mit dem Ziel, diese als oxidische Schichten auf Substraten nach dem CVD-Verfahren abzuscheiden.

Gelöst wird diese Aufgabe durch mehrfunktionelle Tertiäralkohole mit der allgemeinen Formel

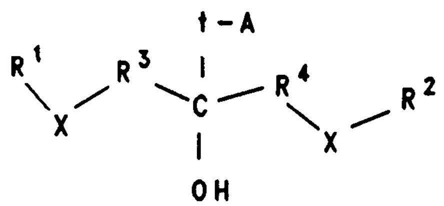

(I)

in der

t-A eine tertiäre Alkylgruppe wie t-Butyl, t-Amyl-, t-Hexyl- oder t-Heptyl-;

$R^1$ und $R^2$ einen geradkettigen oder verzweigten Alkylrest mit 1 bis 7 C-Atomen oder die Gruppe $-[-(CH_2)_nO-]_mR^5$ in der n und m unabhängig voneinander eine ganze Zahl von 1 bis 3 und $R^5$ eine geradkettige oder verzweigte Alkylgruppe mit 1 bis 5 C-Atomen bedeuten,

$R^3$ und $R^4$ einen geradkettigen oder verzweigten Alkylenrest mit 1 bis 4 C-Atomen; und

X eine heteroatomhaltige Gruppe wie -O-, $>N-CH_3$, $>N-C_2H_5$ oder $>N-C_3H_7$ bedeuten.

Verbindungen dieser Art sind bislang in der Literatur noch nicht beschrieben worden und lassen sich nach dem nachstehend beispielhaft aufgeführten Reaktionsschema über Grignard-Reaktionen synthetisieren:

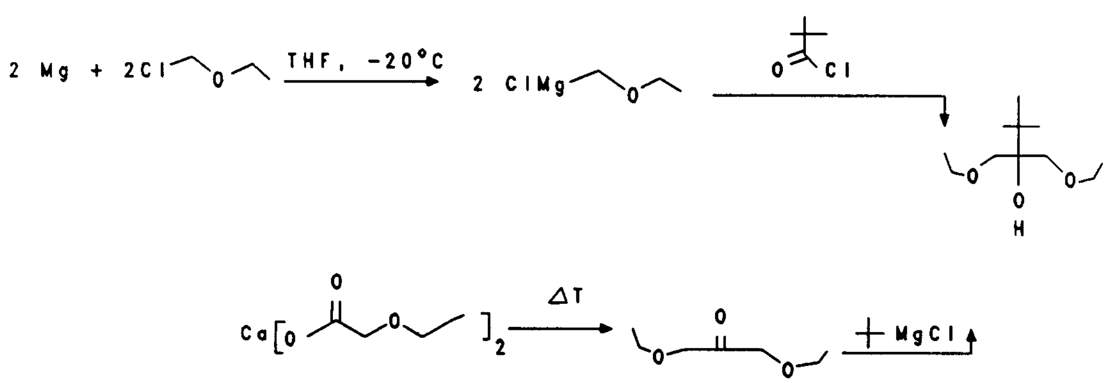


In bevorzugter Ausführungsform stellen die Symbole X ein Sauerstoffatom und die Symbole $R^1$ und $R^2$ Ethyl-, Isopropyl- oder 2-Methoxyethylreste dar.

Die erfindungsgemäßen Tertiäralkohole lassen sich leicht zu reaktiven Zwischenverbindungen umsetzen, z.B. zu den Alkalimetallalkoxiden, und reagieren dann mit Erdalkalisalzen, wie z.B. $BaI_2$, durch Ummetallierung. Man kann die erfindungsgemäßen flüchtigen Erdalkalimetallkomplexe aber auch alternativ durch Umsetzung der freien mehrfunktionellen Tertiäralkohole mit entweder den Erdalkalimetallen selbst (Ca,Sr,Ba) oder mit deren Amiden oder Hydriden herstellen. Besonders bevorzugt sind dabei die Bis-(trimethylsilyl)amide der Formel: $E\{N[Si(CH_3)_3]_2\}_2$ (E = Ca,Sr,Ba), die auch als Solvensaddukte, z.B. mit Tetrahydrofuran (THF), eingesetzt werden können. Diese Herstellungsvarianten sind nachfolgend exemplarisch für Barium dargestellt:

$$BaI_2 + 2K[OC(t\text{-}But)(CH_2OEt)_2] \xrightarrow[-2\ KI]{THF}$$

$$Ba + 2\ HOC(t\text{-}But)(CH_2OEt)_2 \xrightarrow[-\ H_2]{THF} Ba[OC(t\text{-}But)(CH_2OEt)_2]_2$$

$$BaL_2 + 2\ HOC(t\text{-}But)(CH_2OEt)_2 \xrightarrow[-2\ LH]{Pentan}$$

$L = N[Si(CH_3)_3]_2$ oder H

oder anionischer Kohlenwasserstoff-Ligand.

Je nach der Konstitution des gewählten Alkoholats können diese Erdalkalimetallkomplexe in der einfachen Summenformel oder assoziiert zu dimeren oder oligomeren Einheiten vorliegen, wobei natürlich die Flüchtigkeit in dieser Reihenfolge abnimmt.

Die flüchtigen Komplexe bilden farblose, nicht übermäßig luft- und feuchtigkeitsempfindliche Kristalle, die sich in unpolaren Lösungsmitteln, wie z.B. n-Pentan, lösen. Die besondere Eigenschaft der Komplexe ist jedoch ihre hohe Flüchtigkeit, die sich in den typischen Sublimationsdaten, 80 bis 150 °C bei $10^{-2}$ Torr, ausdrückt. Dadurch sind sie für den bestimmungsgemäßen Einsatzzweck als Precursoren für das MO-CVD-Verfahren besonders geeignet. Als Erdalkalimetalle eignen sich neben Barium insbesondere auch Strontium und Calzium.

Durch die nachfolgenden Ausführungsbeispiele soll die Erfindung noch deutlicher beschrieben werden, ohne aber auf die konkret dargestellten Ausführungsformen beschränkt zu sein. Im Rahmen der Beispiele wurden alle Arbeiten in ausgeheizten Glasapparaturen unter absolut strengem Ausschluß von Luft und Feuchtigkeit durchgeführt (Glovebox- und Vakuumline-Techniken). $BaI_2$ wurde vor Gebrauch mehrere Stunden evakuiert, um eventuelle Spuren elementaren Jods zu entfernen. KH (Aldrich) wurde bis zur Farblosigkeit mit n-Hexan gewaschen. $Ba[N(SiMe_3)_2]_2 \cdot 2THF$ wurde dargestellt wie von B.A. Voortstra et al. in Inorg. Chem., Bd. 30, Jahrg. 1991, S. 121-125, beschrieben.

Beispiel 1

Herstellung von 1,3-Diethoxy-2(2,2-dimethylethyl)-2-propanol:

In einem 500 ml-Dreihalskolben mit Rückflußkühler werden 7,40 g (0,304 mol] Magnesium und eine Spatelspitze $HgCl_2$ vorgelegt. Das Gemenge wird mit ungefähr 150 ml THF überschichtet. Zur Aktivierung des Magnesiums wird die Suspension kurz auf ungefähr 50 °C erwärmt (Mg verfärbt sich dunkler) und dann wieder auf Raumtemperatur abgekühlt. In einen 50 ml-Tropftrichter werden 24 ml (25 ml = 0,260 mol) Chlormethylethylether kanüliert (Vorsicht, extrem giftig!) und mit THF auf 50 ml ergänzt. 1 ml reiner Chlormethylethylether werden bei Raumtemperatur direkt zur Mg-THF-Suspension gegeben und nach kurzer Zeit ist die Grignard-Reaktion gestartet. Hat die Reaktionslösung ungefähr 35 °C erreicht, wird sie sofort auf -20 °C abgekühlt; zugleich beginnt man, die THF-Lösung des Chlormethylethylether unter starkem Rühren zuzutropfen. Während des Zutropfens (= 30 min) läßt man die Temperatur der Reaktionsmischung sich zwischen -20 °C und -15 °C einpendeln. Nach Ende der Zugabe wird noch 3 Stunden bei dieser Temperatur gerührt (leichte Graufärbung). Danach wird der Rückflußkühler und der Tropftrichter

entfernt und der Reaktionsansatz in den Gefrierschrank gestellt (-25 °C!). Nach 2 Tagen (Schwenken der Reaktionsmischung im 2-Stunden-Zeitintervall) hat fast das gesamte Mg (Restmenge 1,1 g) abreagiert. Ungefähr 70 ml (45 mmol) $ClMgCH_2OC_2H_5$-THF-Lösung werden bei -25 °C in einen kühlbaren Schlenkrohr vorgelegt. Dann werden bei -15 °C 4.0 ml (3,17 g, 36 mmol) Pivaloylchlorid, mit Ether ergänzt auf 20 ml, innerhalb von 30 min zugetropft und 48 Stunden bei dieser Temperatur gerührt. Nach Erwärmen der Reaktionslösung von -15 °C auf -5 °C wird auf 50 g Eis gegossen. Danach wird soviel gesättigte $NH_4Cl$-Lösung zugegeben, bis sich gerade alles löst. Die organische Phase wird mit je 30 ml gesättigter $NaHCO_3$ und zweimal Eiswasser gewaschen und die vereinigten Wasserphasen nochmals mit Ether gewaschen. Danach wird die vereinigte organische Phase über $MgSO_4$ getrocknet. Fraktionierte Destillation bei 55 °C/1,5 Torr liefert 1,3-Diethoxy-2-(2,2-dimethylethyl)-2-propanol in 79 % Ausbeute, bezogen auf Pivaloylchlorid.

Die Elementaranalyse ergibt folgende Werte:

| | | |
|---|---|---|
| $C_{11}H_{24}O_3$ (204.31) Ber. | C 64.67 | H 11.84 |
| Gef. | C 64.54 | H 11.94 |

Beispiel 2

Herstellung von Kalium[1,3-diethoxy-2(2,2-dimethylethyl)-2-propanolat]: In der Glovebox wird KH (0,410 g, 10,0 mmol) in einen 100 ml Rundkolben mit Rückflußkühler abgewogen und außerhalb der Box 20 ml THF dazukanüliert. Zu dieser Suspension werden unter Rühren bei -78 °C eine Lösung von 1,3-Diethoxy-2-(2,2-dimethylethyl)-2-propanol (2,09 g, 10,2 mmol) in 20 ml Ether während 30 Minuten zugetropft. Man läßt bis Raumtemperatur erwärmen und rührt danach noch 15 Stunden. Danach wird das überschüssige KH über eine Fritte (G4) abfiltriert. Vom Filtrat wird das Lösungsmittel entfernt und die zurückbleibende Substanz im Vakuum getrocknet. Die Substanz wird aus Ether umkristallisiert und in farblosen Kristallen erhalten (0,920 g, 3,80 mmol, 37,1 % Ausbeute).

Beispiel 3

Herstellung von Barium[1,3-diethoxy-2(2,2-dimethylethyl)-2-propanolat]$_2$ (1): 0,857 g (3,54 mmol) Kaliumalkoxid und 0,691 g $BaI_2$ (1,77 mmol) werden in einer Glovebox zusammen mit einem 100 ml Rundkolben eingewogen. Danach wird auf diese Mischung etwa 50 ml THF aufkondensiert und die entstehende reinweiße Suspension 16 Stunden bei Raumtemperatur gerührt. Sämtliche Lösungsmittel werden im Vakuum entfernt und die Substanz wird 3 Stunden getrocknet. Danach werden etwa 50 ml Pentan aufkondensiert, die Suspension 15 Stunden gerührt und anschließend filtriert. Vom Filtrat werden die Lösungsmittel entfernt. Es ergibt sich 0,546 g (0,502 mmol), 56,8 % Ausbeute) 1 als leicht gelbliches öliges Produkt, das beim Stehen langsam kristallisiert.

Die Elementaranalyse ergab folgende Werte:

| | | |
|---|---|---|
| $C_{44}H_{92}Ba_2O_{12}$(1087.9) Ber. | C 48.58 | H 8.52 |
| Gef. | C 50.61 | H 8.45 |

Beispiel 4

Herstellung von Barium[1,3-diethoxy-2(2,2-dimethylethyl)-2-propanolat]$_2$ (1): In der Glovebox werden 1,081 g (1,80 mmol) $Ba[N(SiMe_3)_2]_2 \cdot 2THF$ und 0,734 g (3,59 mmol) 1,3-Dethoxy-2(2,2-dimethylethyl)-2-propanol zusammen in einen 100 ml Rundkolben abgewogen. Es werden sofort 30 ml Pentan in einer Hochvakuum-Apparatur aufkondensiert. Es geht fast alles in Lösung. Anschließend wird 22 Stunden bei Raumtemperatur gerührt. Von dem wenigen weißen Niederschlag wird abfiltriert und das Lösungsmittel vom leicht gelblichen Filtrat im Vakuum (1,5 Torr) entfernt. Nach 2 Stunden Trocknen bei $10^{-3}$ mbar erhält man einen farblosen hochviskosen Rückstand, der beim Stehen langsam zu 1 kristallisiert (0,930 g, 1,71 mmol, 95 % Ausbeute).

Beispiel 5

Herstellung von Bariumbis[1,3-di(1-methylethoxy)-2(2,2-dimethylethyl)-2-propanolat]:
In der Glove-Box wird zu 2,016 g (3,35 mmol) $Ba[N(SiMe_3)_2]_2 \cdot 2THF$ in 55 ml n-Pentan 1,555 g (6,69 mmol) 1,3-Di(1-methylethoxy)-2(2,2-dimethylethyl)-2-propanol getropft. Anschließend wird 22 Stunden bei Raumtemperatur gerührt. Das Lösungsmittel wird im Vakuum (1,5 Torr) entfernt. Nach 9 Stunden Trocknen bei $10^{-3}$ mbar erhält man einen farblosen hochviskosen Rückstand, der beim Stehen langsam kristalliert (1,977 g, 3,29 mmol, 98,4 % Ausbeute). Die Verbindung sublimiert quantitativ bei 170 °C/$10^{-2}$ Torr.

Beispiel 6

Herstellung von Bariumbis[1,3-di(2-methoxyethoxy)-2(2,2-dimethyethyl)-2-propanolat]:
In der Glove-Box wird zu 0,253 g (0,42 mmol) $Ba[N(SiMe_3)_2]_2 \cdot 2THF$ in 40 ml n-Pentan 0,222 g (0,84 mmol) 1,3-Di(2-methoxyethoxy)-2(2,2-dimethylethyl)-2-propanol getropft. Anschließend wird 22 Stunden bei Raumtemperatur gerührt.

Das Lösungsmittel wird im Vakuum (1,5 Torr) entfernt. Nach 9 Stunden Trocknen bei $10^{-3}$ mbar erhält man einen farblosen hochviskosen Rückstand, (0,245 g, 0,37 mmol, 88 % Ausbeute). Die Verbindung sublimiert quantitativ bei 90 °C/$10^{-2}$ Torr.

## Patentansprüche

1. Mehrfunktionelle Tertiäralkohole geeignet als Precursoren für die Auftragung von Metalloxiden nach dem Verfahren der chemical vapor deposition, dadurch gekennzeichnet, daß sie die allgemeine chemische Formel:

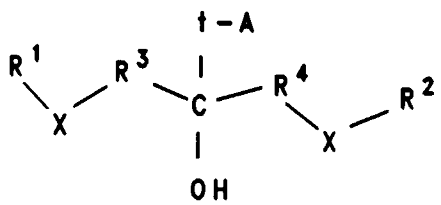

(I)

besitzen, in der

| | |
|---|---|
| t-A | eine tertiäre Alkylgruppe wie t-Butyl, t-Amyl-, t-Hexyl- oder t-Heptyl-; |
| $R^1$ und $R^2$ | einen geradkettigen oder verzweigten Alkylrest mit 1 bis 7 C-Atomen oder die Gruppe $-[(CH_2)_nO-]_mR^5$ in der n und m unabhängig voneinander eine ganze Zahl von 1 bis 3 und $R^5$ eine geradkettige oder verzweigte Alkylgruppe mit 1 bis 5 C-Atomen bedeuten, |
| $R^3$ und $R^4$ | einen geradkettigen oder verzweigten Alkylenrest mit 1 bis 4 C-Atomen; und |
| X | eine heteroatomhaltige Gruppe wie -O-, $>N-CH_3$, $>N-C_2H_5$ oder $>N-C_3H_7$ bedeuten. |

2. Mehrfunktionelle Tertiäralkohole nach Anspruch 1, dadurch gekennzeichnet, daß die Symbole t-A eine tertiäre Butylgruppe, X ein Sauerstoffatom und $R^1$ und $R^2$ geradkettige Reste mit 1 bis 3 C-Atomen oder die Gruppe $-CH_2CH_2OCH_3$ darstellen.

3. Flüchtige Komplexe von Erdalkalimetallen mit mehrfunktionellen Tertiäralkoholen nach Anspruch 1 oder 2 erhalten durch Ummetallierung der Alkalimetallalkanolate.

4. Flüchtige Komplexe von Erdalkalimetallen mit mehrfunktionellen Tertiäralkoholen nach Anspruch 1 oder 2 erhalten durch Umsetzung der Alkalimetallalkanolate mit Salzen der Erdalkalimetalle.

5. Flüchtige Komplexe von Erdalkalimetallen mit mehrfunktionellen Tertiäralkoholen nach Anspruch 1 oder 2 erhalten durch direkte Umsetzung der Erdalkalimetalle mit den Alkoholen.

6. Flüchtige Komplexe von Erdalkalimetallen mit mehrfunktionellen Tertiäralkoholen nach Anspruch 1 oder 2 erhalten durch Alkoholyse von Erdalkalimetallamiden und/oder -hydriden.

7. Flüchtige Komplexe nach einem der Ansprüche 3 bis 6, dadurch gekennzeichnet, daß sie als Metalle Barium, Strontium oder Calcium enthalten.

8. Verwendung von flüchtigen Komplexen nach einem der Ansprüche 3 bis 7 als Precursoren zur Herstellung von Metalloxidschichten durch Abscheidung aus der Gasphase.

Europäisches Patentamt

# EUROPÄISCHER RECHERCHENBERICHT

Nummer der Anmeldung
EP 93 11 7746

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | CH-A-527 143 (UGINE KUHLMANN)<br>* Spalte 2, Verbindung IV *<br>--- | 1 | C07C43/13<br>C23C16/18<br>C07C215/18 |
| A | DE-C-173 610 (FARBENFABRIKEN)<br>* Beispiel 1 *<br>--- | 1 | |
| A | US-A-4 558 144 (R.C. FAY ET AL.)<br>* Spalte 2, Zeilen 41-52 *<br>--- | 1 | |
| A | US-A-5 064 686 (M.J. MCGEARY)<br>* Anspruch 1 *<br>----- | 1 | |

RECHERCHIERTE SACHGEBIETE (Int.Cl.5)
C07C
C23C

Der vorliegende Recherchenbericht wurde für alle Patentansprüche erstellt

| Recherchenort | Abschlußdatum der Recherche | Prüfer |
|---|---|---|
| BERLIN | 16. Februar 1994 | Probert, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C00)